# EUROPEAN PATENT SPECIFICATION

(11) **EP 3 981 462 B1**
(45) Date of publication and mention of the grant of the patent: **02.12.2026**
(21) Application number: 21201784.2
(22) Date of filing: 08.10.2021
(51) Int. Cl.: A61M 27/00, A61M 25/00

(54) **INCREASED DRAINAGE AND DECREASED INTERNAL STRESS URETERAL STENT DESIGN**
ENTWURF EINES HARNLEITER-STENTS MIT ERHÖHTER DRAINAGE UND VERRINGERTER INNERER SPANNUNG
CONCEPTION D'ENDOPROTHÈSE URÉTÉRALE À DRAINAGE ACCRU ET STRESS INTERNE RÉDUIT

(30) Priority: 08.10.2020 US 202063089149 P
(43) Date of publication of application: 13.04.2022
(73) Proprietor: Cook Medical Technologies LLC, Bloomington, IN 47404 (US)
(72) Inventor: CHANDRA, Santanu, West Lafayette, 47906 (US); SWIFT, Richard, South Bend, 46635 (US); HEFFERNAN, Conor, Limerick, V94PEV9 (IE); KILLEY, Nathan, Appleton, 54915 (US); SHEETS, Jonathan, Greenwood, 46143 (US)
(74) Representative: Leach, Sean Adam

(56) References cited:
- CN-A- 107 510 878
- CN-U- 208 065 305
- CN-U- 211 096 882
- US-A- 5 599 291
- US-A1- 2002 188 246
- US-A1- 2017 348 512
- US-A1- 2019 105 474

## Description

### TECHNICAL FIELD

Embodiments disclosed herein generally relate to stents, and more particularly to stents configured for use in and through a ureter.

### BACKGROUND

Ureteral stents are widely used prior to and/or after various urological procedures and serve to provide drainage of urine from the kidney into the bladder with an indwell time of up to 12 months. Typically, ureteral stents comprise an elongate member with an internal-luminal space, the member having at least one end coiled in a pigtail-shape, a substantially straight portion, and a plurality of side ports. While the stent is indwelling, urine passes through the internal-luminal space between the ureter and the ureteral stent as well as passing into and out of side ports located along the length of the stent, which allows urine to flow through the stent inner lumen. The size, shape, and location of the side ports dictate the drainage characteristics of the stent. Larger side port diameter and a greater number of side ports may lead to more removal of material that increases the drainage characteristics but may lead to high stress in the device. If the stress is allowed to reach a high enough level, the device may fail, resulting in a delayed procedure, fragmenting of the stent, and the potential for additional intervention. Also, not all the side ports are uniformly active when deployed in the ureter, which leads to stasis regions inside the inactive side ports. These stasis regions may lead to encrustation formations. Therefore, strategic placement of the side ports is crucial in optimization of the flow characteristics and mitigating the risk of encrustations. Furthermore, devices with strategic placement of the side ports may offer reduced manufacturing complexity and cost.

The stent of the current disclosure may rectify the formation of encrustation at side ports locations due to improper drainage characteristics. One objective of the present disclosure is to improve the drainage characteristics by strategic placement of the side ports in the kidney-ureter junction as well as to reduce the internal stress present within the stent during use. The strategic placement includes identification of the high drainage location in a stent after its placement in the human physiology and then increasing the number of side ports in that location of the kidney-ureter and ureter-bladder junctions and reducing the number of side ports in low drainage locations inside the kidney, bladder and ureter.

US 5599291 describes a ureteral stent that comprises an elongated member having a proximal end portion and a distal end portion joined by a body portion.

US 2019/105474 describes a catheter having an elongated tube with a central lumen, a proximal end, and a distal end terminating in a tip; a balloon and a balloon lumen that is in fluid communication with the balloon, the balloon being situated at a point along the elongated tube between the proximal and distal ends of the elongated tube; and a catheter hub that is connected to the proximal end of the elongated tube, the catheter hub being in fluid communication with the central lumen and the balloon lumen. The elongated tube has one or more side holes between the balloon and the tip of the distal end of the elongated tube or an end hole at the tip of the elongated tube. The elongated tube also has one or more side holes between the balloon and the hub.

CN208065305U describes a ureteral stent.

CN107510878A describes a multi cavity ureter support. The multi-cavity ureter support comprises a first curling section, a straight pipe section and a second curling section, the straight pipe section includes a first straight pipe and at least one second straight pipe , the first straight pipe, the first curling section and the second curling section compose a pipe, the second straight pipe is located outside the first straight pipe, and the second straight pipe and the first straight pipe are two independent pipes.

CN211096882U describes a multifunctional ureter support tube.

US2002188246 describes a method of treating the upper urinary tract of a mammal having a urethra extending to a bladder and a ureter extending from the bladder to a kidney.

US2017348512 describes a ureteral catheter that includes a drainage lumen having a proximal portion configured to be positioned in at least a portion of a patient's urethra and/or bladder and a distal portion configured to be positioned in a patient's kidney, renal pelvis, and/or in the ureter adjacent to the renal pelvis.

### BRIEF SUMMARY

An aspect of the invention is set out in the independent claim and optional features are set out in the dependent claims.

The disclosed invention relates generally to a medical device assembly, more specifically to a ureteral stent.

One general aspect of the present disclosure includes a stent comprising an elongate member, the elongate member having a first coil section defining a lumen and comprising a first coil, a first substantially straight section defining a lumen, a second substantially straight section defining a lumen and located between the first coil section and the first substantially straight section, and a plurality of side ports wherein the plurality of side ports are located only in the substantially straight sections.

The lumen extends continuously through the coil(s) and the straight section(s) as well as also being in fluid communication with an exterior of the member through the side ports.

Other features and advantages of the invention will be. or will become, apparent to one with skill in the art upon examination of the following figures and detailed description. It is intended that all such additional systems, methods, features, and advantages be within the scope of the invention, and be encompassed by the following claims.

### BRIEF DESCRIPTION OF DRAWINGS

The present disclosure can be better understood with reference to the following drawings and description. The components in the figures are not necessarily to scale, emphasis instead being placed upon illustrating the principles of the present disclosure. Moreover, in the figures, like-referenced numerals designate corresponding parts throughout the different views.

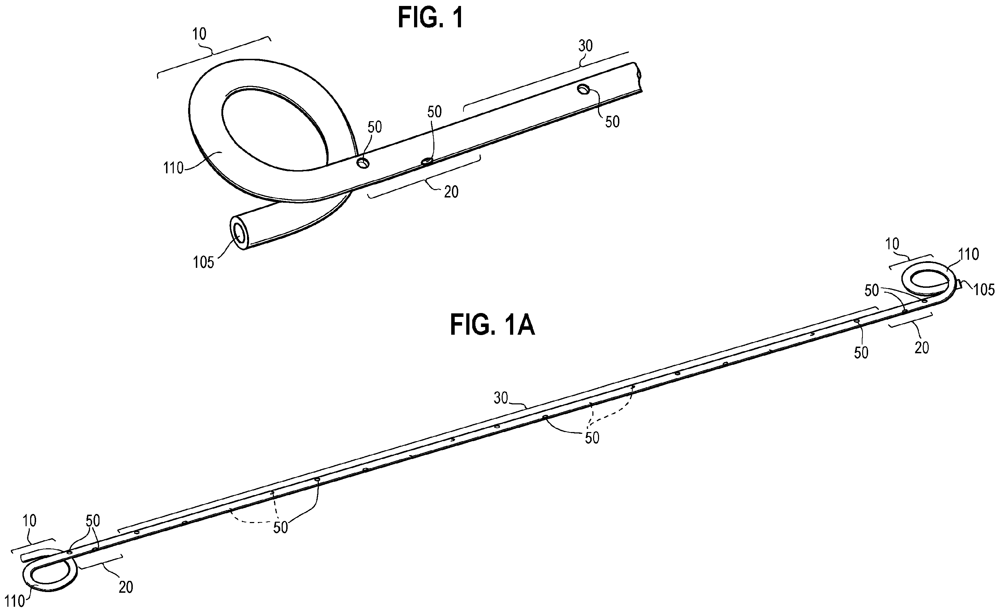

FIG. 1 is an illustration showing a perspective view of one end portion of a stent in accordance with certain aspects of the present disclosure; FIG. 1A is an illustration showing a perspective view of a stent in accordance with certain aspects of the present disclosure (e.g., FIG. 1), and indicating also that the side on which end curls/coils lie can go the same direction or opposite directions in different embodiments.

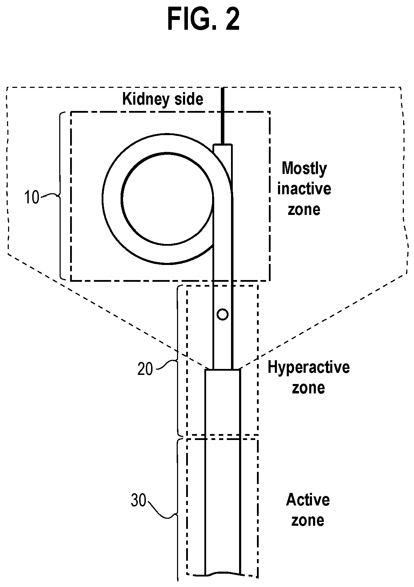

FIG. 2 is an illustration showing relative positions of the device elements within a human body.

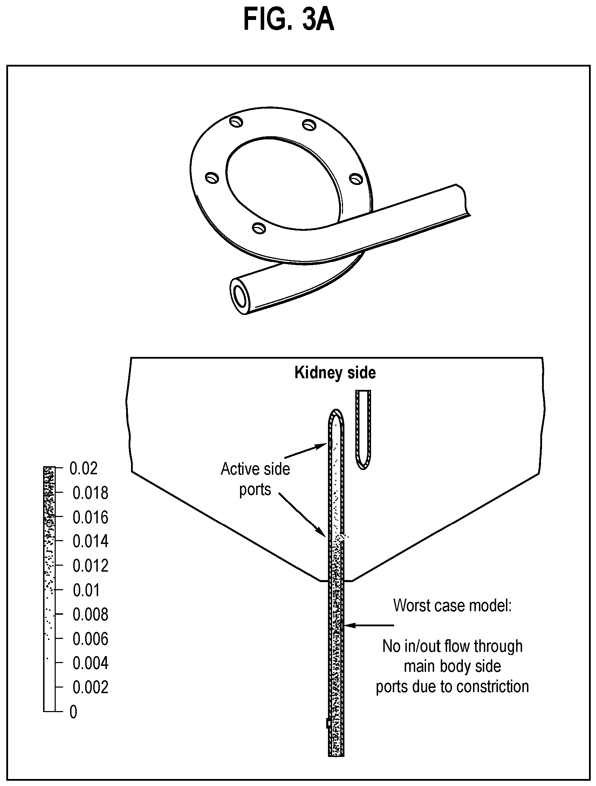

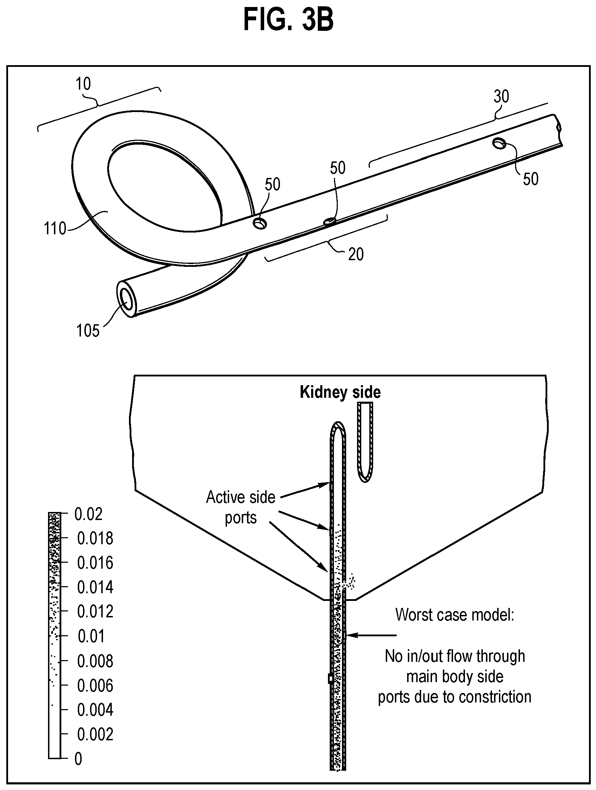

FIGS. 3A-B is a comparison of flow characteristics, FIG. 3A showing flow characteristics of a pigtail stent having side ports in pigtail curl and FIG. 3B showing flow characteristics of a pigtail stent in accordance with certain aspects of the present disclosure.

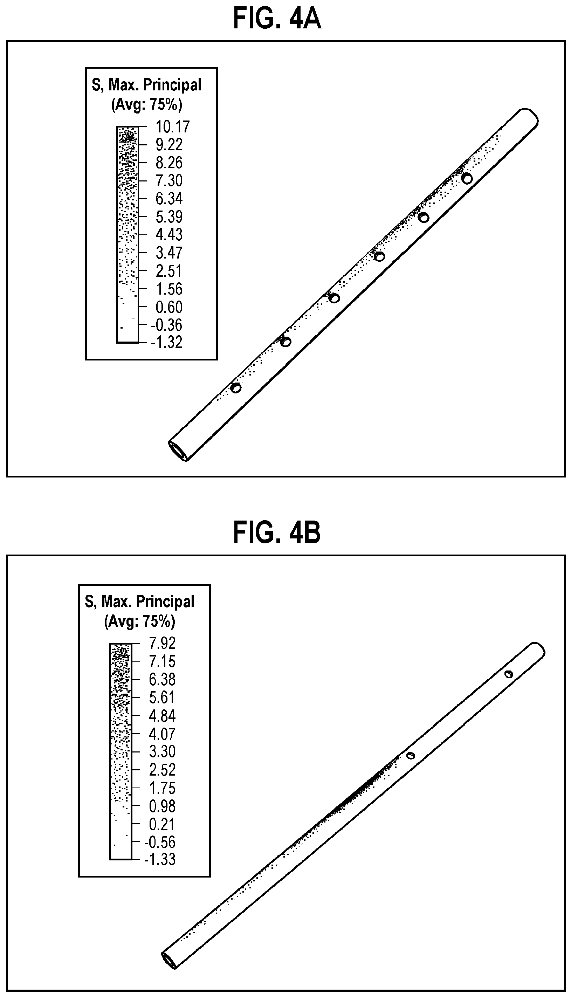

FIGS. 4A-4B show a comparison of ureteral stent stress levels after pigtail unwinding, with FIG. 4A showing stress level of a pigtail stent having side ports along the coil of the pigtail section and FIG. 4B showing stress level of a pigtail stent in accordance with certain aspects of the present disclosure like the embodiments of FIGS. 1-1A.

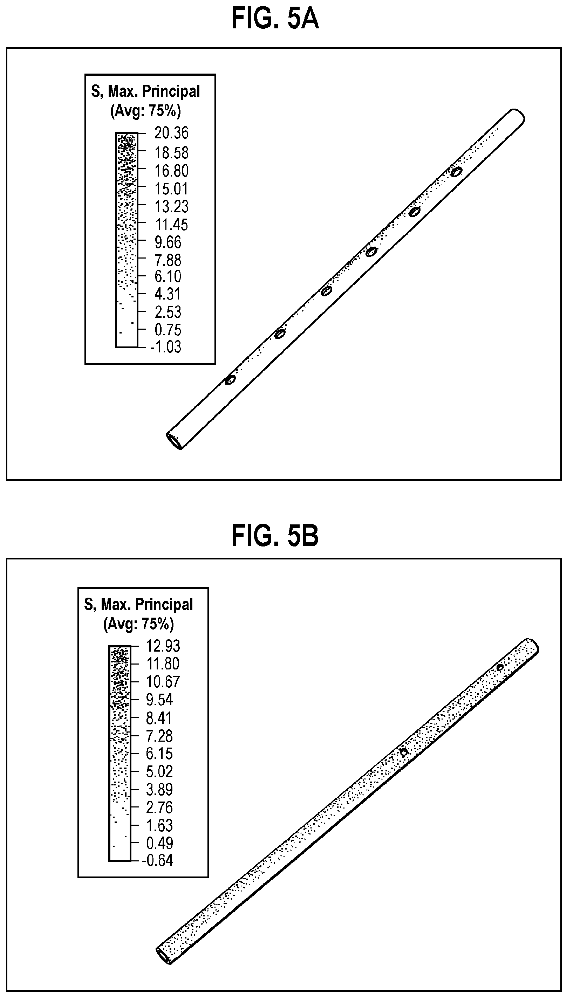

FIG. 5 is a comparison of ureteral stent stress levels after pigtail unwinding and extraction force application, FIG. 5A showing stress level of a pigtail stent having side ports on pigtail section and FIG. 5B showing stress level of a pigtail stent in accordance with certain aspects of the present disclosure.

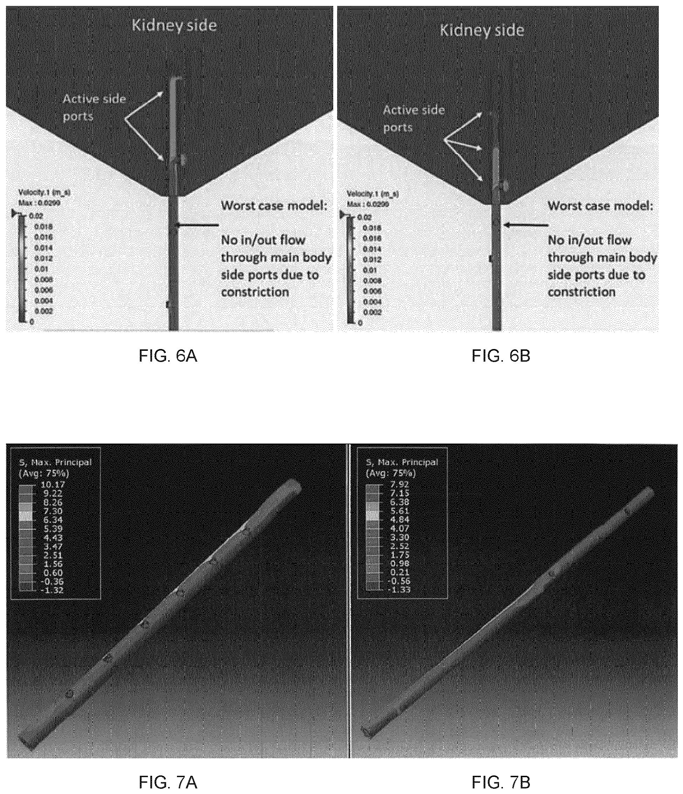

FIG. 6A and FIG. 6B comprise the original colour images upon which the drawings in FIG. 3A and FIG. 3B respectively are based in that FIG. 6A shows the same flow characteristics of a pigtail stent having side ports in pigtail curl as FIG 3A and FIG. 6B shows the same flow characteristics of a pigtail stent in accordance with certain aspects of the present disclosure as in FIG 3B.
FIG. 7A and FIG. 7B comprise the original colour images upon which the drawings in FIG. 4A and FIG. 4B respectively are based in that FIG. 7A shows the same stress level of a pigtail stent having side ports along the coil of the pigtail section as FIG. 4A and FIG. 7B shows the same stress level of a pigtail stent in accordance with certain aspects of the present disclosure like the embodiments of FIGS. 1-1A as in FIG. 4B.

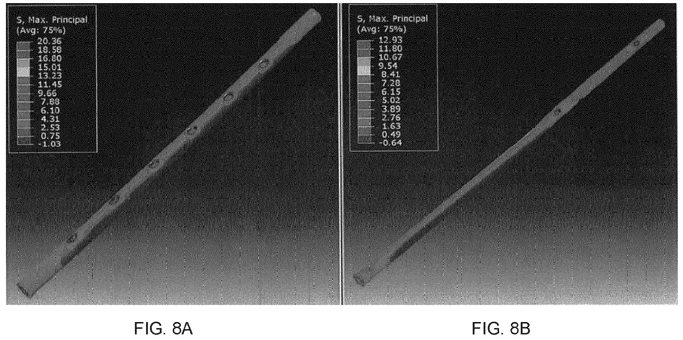

FIG. 8A and FIG. 8B comprise the original colour images upon which the drawings in FIG. 5A and FIG. 5B respectively are based in that FIG. 8A shows the same stress level of a pigtail stent having side ports on pigtail section as in FIG. 5A and FIG. 8B shows stress level of a pigtail stent in accordance with certain aspects of the present disclosure as in Fig. 5B.

### DETAILED DESCRIPTION

Various aspects are described below with reference to the drawings in which like elements generally are identified by like numerals. The relationship and functioning of the various elements of the aspects may better be understood by reference to the following detailed description. However, aspects are not limited to those illustrated in the drawings or explicitly described below. It also should be understood that the drawings are not necessarily to scale, and in certain instances details may have been omitted that are not necessary for an understanding of aspects disclosed herein, such as conventional material, construction, and assembly. The terms "about," "substantially," "generally," and other terms of degree, when used with reference to any volume, dimension, proportion, or other quantitative or qualitative value, are intended to communicate a definite and identifiable value within the standard parameters that would be understood by one of skill in the art (equivalent to a medical device engineer with experience in this field), and should be interpreted to include at least any legal equivalents, minor but functionally-insignificant variants, standard manufacturing tolerances, and including at least mathematically significant figures (although not required to be as broad as the largest range thereof). For example, the term "substantially straight" means at least more nearly straight than curved, but generally and preferably means as nearly straight as possible within realistic physical constraints of the materials used and manufacturing tolerances therefor.

Referring to FIGS. 1-1A, a pigtail stent 100 may include an elongate tubular member 110 with an internal-luminal space 105, the member 110 having at least one pigtail curl 10 (alternatively called a coil, as it forms a nearly helical loop) at an end, a substantially straight portion near the beginning of the pigtail curl 20, a substantially straight portion of the stent body 30, and a plurality of side ports 50. Substantially straight portions 20, 30 refer to portions of the stent 100 that establishes a substantially straight profile in a nominal direction, which need not be perfectly linear. Because stent 100 is generally made of a flexible material, the substantially straight portions 20, 30 may follow an arcuate lumen deployed therein. As should be understood with reference to FIG. 1A, the stent 100 may actually be generally symmetrical in gross construction (e.g., double pigtailed) and/or in fine detail (e.g., number and placement of side ports), where a double-pigtail embodiment may have the first and coils at opposite ends matching or mirroring each other along or across any axis of the device and including a second coiled portion 10 and third substantially straight portion 20, at the opposite end of the first substantially straight portion 30. FIG. 2 illustrates relative positions of the curl 10, the second substantially straight portion 20 near the beginning of the curl 10, and the substantially straight portion of the body 30 inside a human body. When disposed in a human body, the first pigtail curl 10 may be located inside kidney whereas the substantially straight portions 20, 30 may generally be located in/ through a ureter, and a second pigtail curl 10 (if present) may be located in the bladder of the body where the device is installed. Typical stent dimensions may be between 5 Fr-9 Fr (about 1.66 to about 3 mm in diameter) and about 15 to about 35 cm in length including one or two coiled pigtails (meaning that the uncoiled length would be greater)

In one embodiment of the invention, the stent 100 has a plurality of side ports 50 only on substantially straight portions 20, 30, with no side ports along/around the coil 10 of the pigtail. Specifically, side ports 50 on the substantially straight portion near the beginning of the pigtail curl 10 may be spaced 0.5 cm apart, with adjacent side ports 50 lying along an axis that is rotated/offset 90° from the other side ports 50 along the body of the substantially straight portion. The number of side ports 50 in the substantially straight portion 20 near the beginning of the pigtail curl 10 determines the drainage. For example, increasing the number of side ports 50 in the substantially straight portion 20 near the beginning of the pigtail curl 10 may result in greater drainage with the same flow rate through the lumen within which the stent is deployed, such as the ureter. Thus, the number of side ports 50 in the substantially straight portion near the beginning of the pigtail curl 10 may vary depending on the desired drainage characteristics. In one embodiment, the number of side ports 50 in the substantially straight portion 20 near the beginning of the pigtail curl 10 may be five, while in other embodiments it may be alternative numbers, such as 3-10 ports, inclusive of all numbers within this range. Additionally, side ports 50 in the substantially straight portion of the body 30 may be spaced apart at various longitudinal distances. For example, side ports 50 in the substantially straight portion of the body 30 may be longitudinally spaced at least up to 5 mm or 1 cm apart from each other, or further apart. The side ports 50 may be, but do not need to be, uniformly spaced longitudinally apart from each other. For example, the side ports 50 may be longitudinally spaced about 5 mm from each other along the substantially straight portion 20, and then spaced further apart along the middle substantially straight portion 30. For another example, side ports 50 in the substantially straight portion of the body 30 may be spaced 3 cm apart from each other. The side ports 50 in the substantially straight portion of the body 30 may be positioned upon the portion such that the side ports are positioned at/ offset by 90° angles between adjacent, longitudinally separated side ports along the length of the substantially straight portion. In other embodiments, there may be different angles between adjacent side ports, such as 30, 45, 60, or 75 degrees. Stated differently, each of the plurality of side ports on the second substantially straight section is offset rotated (e.g., 90° or some other amount of about 30°-90°) relative to each adjacent side port, such that they are disposed along a spiral or helix where each side port is located radially and longitudinally offset relative to each longitudinally-adjacent side port.

The drainage along the substantially straight portions 20, 30 of the stent can be characterized by calculations of the flow resistance, which is a ratio of pressure gradient and flow rate at inlet. The pressure gradient is defined as the pressure difference between inlet and outlet. Not every one of the side ports is uniformly active when deployed in the ureter. However, proper drainage characteristics are important because stasis regions, which may be formed inside inactive side ports, may lead to encrustation formations. Referring to FIGS. 3A-3B, a device constructed in accordance with certain aspects of the present disclosure (e.g., as shown in FIG. 1 and the upper portion of FIG. 3A) exhibited a reduced flow resistance of the device by 10% for a flow rate of 1 mL/min compared a device having side ports along the coiled pigtail portion (as shown in the upper portion of FIG. 3B). Specifically, FIG. 3A shows a colorimetric diagram with flow characteristics of a device having side ports along the coiled pigtail portion. The device having side ports along the pigtail portion exhibit a substantial decrease in drainage inside kidney through the most of the side ports in the pigtail region. One side port around the junction of kidney and bladder region, which is equivalent to the substantially straight portion near the beginning of pigtail curl, is hyperactive. Therefore, FIG. 3A flow diagram shows two active side ports as in a prior art catheter. On the other hand, FIG. 3B shows a colorimetric diagram with flow characteristics of the device in accordance with certain aspects of the present disclosure having more side ports around the junction of kidney and bladder region but having no side ports along the coiled pigtail portion. A device constructed in accordance with certain aspects of the present disclosure exhibits gradual increase in drainage in the kidney-ureter junction region, and thus has more side ports that are relatively active as compared to prior art stents that have side ports along the coiled pigtail. FIG. 3B shows three active side ports. Eliminating the side ports on the pigtail curl and increasing the number of side ports on the substantially straight portion near the beginning of pigtail curl in accordance with certain aspects of the present disclosure reduces the number of stasis locations inside the inactive side ports, thereby reducing the risk for encrustation.

Referring to FIGS. 4A-4B, the device in accordance with certain aspects of the present disclosure exhibited a decreased stress level during the straightening of the pigtail curl 10, which is identified as one of the highest stress scenarios experienced by a stent during use. FIG. 4A shows a colorimetric representation of stress levels of the straightened pigtail portion of a device having side ports linearly along the pigtail curl whereas FIG. 4B shows stress levels of the straightened pigtail portion of the device having no side ports along the pigtail portion in accordance with certain aspects of the present disclosure. Stress levels along the straightened portion of the device constructed in accordance with certain aspects of the present disclosure were generally lower than that of the device having side ports along the pigtail portion (e.g., as shown in the upper portion of FIG. 3A). Particularly, the decrease in stress level was of 28% compared to that of the pigtail stent having side ports on the coiled pigtail portion. This presents advantages for the device that will readily be appreciated by those of skill in the art, including with regard to maintaining device integrity and/or to minimizing stresses applied to patient tissues during installation and removal of a stent constructed in keeping with this disclosure.

Referring to FIGS. 5A-5B, the device in accordance with certain aspects of the present disclosure exhibited a decreased peak stress during exaggerated tensile extension of the stent, which reflects the worst-case expected loading scenario during stent retrieval, when the coiled pigtail portion may be forced out of its coil to be substantially or absolutely straight. Specifically, FIG. 5A shows a colorimetric diagram of the stress level of a device having side ports linearly along the pigtail portion (e.g., as shown in the upper portion of FIG. 3A), whereas FIG. 5B shows a colorimetric diagram of the stress level of the device having no side ports along the pigtail portion in accordance with the present disclosure (e.g., as shown in FIG. 1). Stress levels of the straightened portion of the device in accordance with certain aspects of the present disclosure were markedly lower than that of the device having side ports along the coiled pigtail portion. Specifically, the decrease was 57% compared to that of the pigtail stent having side ports on the pigtail portion.

In addition to improved performance characteristics, devices without side ports in the curled pigtail region in accordance with certain aspects of the present disclosure offer generally reduced manufacturing complexity and cost. Those factors - along with the above-described changes in stresses during device life and usage, and in improved flow with corresponding reduced risk of encrustation that would diminish device efficacy- demonstrate the criticality of the present inventive features.

The stent of the present disclosure may be ureteral stent, such as those described herein. The elongate member has an internal lumen, the member has at least one coil section, which may be in a pigtail-shape. The coil also provides an internal lumen which is a coil shape defined by the walls of the coil section of the elongate member. The internal lumen of the coil section(s) may be continuous with the internal lumen of the rest of the elongate member such as the straight section(s). The walls of the elongate member may comprise a material for inhibiting the ingress and egress of liquid to and from the internal-lumenal space, for example it may be a continuous e.g., sheet-like material, such as plastic.

While the preferred embodiments of the disclosure have been described, it should be understood that the disclosure is not so limited and modifications may be made without departing from the scope of the present disclosure. The scope of the invention is defined by the appended claims, and all devices that come within the meaning of the claims, either literally or by equivalence, are intended to be embraced therein.

## Claims

1. A stent (100) comprising:
an elongate tubular member (110) comprising:
a first coil section defining a lumen and comprising a first coil (10);
a second coil section comprising a second coil (10);
a first substantially straight section (30) through which the lumen continues;
a second substantially straight section (20) located between the first coil section and the first substantially straight section;
a third substantially straight section (20) located between the second coil section and the first substantially straight section ; and
a plurality of side ports (50) wherein the plurality of side ports is located only along the substantially straight sections, where the lumen extends continuously through the first coil, the first, second, and third substantially straight sections, and the second coil, as well as also communicating with an exterior of the member through the side ports,
wherein at least some of the plurality of side ports (50) are more closely spaced along the second and third substantially straight sections (20) than along the first substantially straight section (30).

2. The stent of claim 1, wherein each of the plurality of side ports along the third substantially straight section is offset rotated from 30° to 90° relative to each adjacent side port.

3. The stent of claim 1 or 2, wherein at least some of the plurality of side ports are uniformly spaced away from each other.

4. The stent of any of claims 1 to 3, wherein the plurality of side ports along the second substantially straight section includes three to ten side ports.

5. The stent of any of claims 1 to 4, wherein at least some of the plurality of side ports are spaced away from each other by about 5 mm to about 3 cm.

## Patentansprüche

1. Stent (100), umfassend:
ein längliches röhrenförmiges Element (110), umfassend:
einen ersten Wendelabschnitt, der ein Lumen definiert und eine erste Wendel (10) umfasst;
einen zweiten Wendelabschnitt, der eine zweite Wendel (10) umfasst;
einen ersten im Wesentlichen geraden Abschnitt (30), durch den sich das Lumen fortsetzt;
einen zweiten im Wesentlichen geraden Abschnitt (20), der sich zwischen dem ersten Wendelabschnitt und dem ersten im Wesentlichen geraden Abschnitt befindet;
einen dritten im Wesentlichen geraden Abschnitt (20), der sich zwischen dem zweiten Wendelabschnitt und dem ersten im Wesentlichen geraden Abschnitt befindet; und
eine Mehrzahl von Seitenöffnungen (50), wobei sich die Mehrzahl von Seitenöffnungen nur entlang der im Wesentlichen geraden Abschnitte befindet, wobei sich das Lumen kontinuierlich durch die erste Wendel, den ersten, zweiten und dritten im Wesentlichen geraden Abschnitt und die zweite Wendel erstreckt sowie auch über die Seitenöffnungen mit einer Außenseite des Elements in Verbindung steht,
wobei mindestens einige der Mehrzahl von Seitenöffnungen (50) entlang des zweiten und dritten im Wesentlichen geraden Abschnitts (20) enger beabstandet sind als entlang des ersten im Wesentlichen geraden Abschnitts (30).

2. Stent nach Anspruch 1, wobei jede der Mehrzahl von Seitenöffnungen entlang des dritten im Wesentlichen geraden Abschnitts um 30° bis 90° relativ zu jeder benachbarten Seitenöffnung drehversetzt ist.

3. Stent nach Anspruch 1 oder 2, wobei mindestens einige der Mehrzahl von Seitenöffnungen gleichmäßig voneinander beabstandet sind.

4. Stent nach einem der Ansprüche 1 bis 3, wobei die Mehrzahl von Seitenöffnungen entlang des zweiten im Wesentlichen geraden Abschnitts drei bis zehn Seitenöffnungen beinhaltet.

5. Stent nach einem der Ansprüche 1 bis 4, wobei mindestens einige der Mehrzahl von Seitenöffnungen um etwa 5 mm bis etwa 3 cm voneinander beabstandet sind.

## Revendications

1. Endoprothèse (100) comprenant :
un élément tubulaire allongé (110) comprenant :
une première section de bobine définissant une lumière et comprenant une première bobine (10) ;
une seconde section de bobine comprenant une seconde bobine (10) ;
une première section sensiblement droite (30) à travers laquelle la lumière continue ;
une deuxième section sensiblement droite (20) située entre la première section de bobine et la première section sensiblement droite ;
une troisième section sensiblement droite (20) située entre la seconde section de bobine et la première section sensiblement droite ; et
une pluralité d'orifices latéraux (50), la pluralité d'orifices latéraux étant située uniquement le long des sections sensiblement droites, la lumière s'étendant de manière continue à travers la première bobine, les première, deuxième et troisième sections sensiblement droites, et la seconde bobine, ainsi qu'en communiquant également avec un extérieur de l'élément à travers les orifices latéraux,
au moins certains orifices latéraux (50) étant plus étroitement espacés le long des deuxième et troisième sections sensiblement droites (20) que le long de la première section sensiblement droite (30).

2. Endoprothèse selon la revendication 1, chacun de la pluralité d'orifices latéraux le long de la troisième section sensiblement droite étant décalé en rotation de 30° à 90° par rapport à chaque orifice latéral adjacent.

3. Endoprothèse selon la revendication 1 ou 2, au moins certains de la pluralité d'orifices latéraux étant espacés uniformément les uns des autres.

4. Endoprothèse selon l'une quelconque des revendications 1 à 3, la pluralité d'orifices latéraux le long de la deuxième section sensiblement droite comprenant trois à dix orifices latéraux.

5. Endoprothèse selon l'une quelconque des revendications 1 à 4, au moins certains de la pluralité d'orifices latéraux étant espacés les uns des autres d'environ 5 mm à environ 3 cm.
